# EUROPEAN PATENT APPLICATION

(11) **EP 4 104 887 A1**
(43) Date of publication of application: **21.12.2022**
(21) Application number: 22176136.4
(22) Date of filing: 30.05.2022
(51) Int. Cl.: A61M 25/00, A61B 1/005, A61B 1/012, A61B 1/05

(54) **DELIVERY SYSTEM WITH DUAL LUMEN CATHETER**

(30) Priority: 11.06.2021 US 202163209689 P
(71) Applicant: Cook Medical Technologies LLC, Bloomington, IN 47404 (US)
(72) Inventor: BENN, Niall, Castleconnell (IE); O'MALLEY, Jennifer, Mungret (IE); ROSCA, Inga, Corbally (IE); COLLINS, Jack, Cratloe (IE)
(74) Representative: Leach, Sean Adam

(57) **Abstract**

A system for delivering an agent including a plurality of particles to a target site. The system incudes a catheter (40) having a first wall defining an inner lumen (44) for delivery of the agent to the target site, and a second wall surrounding the first wall to define an outer lumen (45) therebetween for delivery of a fluid to the target site. The catheter has a distal end terminating with a primary opening of the inner lumen and a secondary opening of the outer lumen. A hub (56) associated with a proximal end of the catheter (40) includes a first inlet (58) for receiving a supply of the agent and a second inlet (60) for receiving a supply of the fluid, wherein the first inlet (58) is in fluid communication with the inner lumen (44), and the second inlet (60) is in fluid communication with the outer lumen (45) .

## Description

### RELATED APPLICATIONS

This application claims the benefit of U.S. Provisional Application No. 63/209,689, filed on June, 11, 2021, pending, the entirety of which is incorporated herein by reference.

### BACKGROUND

The present embodiments relate generally to medical devices, and more particularly, to systems and methods for delivering therapeutic agents to a target site while maintaining suitable visibility of the target site and surrounding environment.

There are several instances in which it may become desirable to introduce therapeutic agents into the human or animal body. For example, therapeutic drugs or bioactive materials may be introduced to achieve a biological effect. The biological effect may include an array of targeted results, such as inducing hemostasis, sealing perforations, reducing restenosis likelihood, or treating cancerous tumors or other diseases.

Many of such therapeutic agents are injected using an intravenous (IV) technique and via oral medicine. While such techniques permit the general introduction of medicine, in many instances it may be desirable to provide localized or targeted delivery of therapeutic agents, which may allow for the guided and precise delivery of agents to selected target sites. For example, localized delivery of therapeutic agents to a tumor may reduce the exposure of normal, healthy tissues to the therapeutic agents, which may reduce potentially harmful side effects.

Localized delivery of therapeutic agents has been performed using catheters and similar introducer devices. By way of example, a catheter may be advanced to a target site within the patient, where the therapeutic agent may be sprayed or ejected from the catheter into the target site. Typically, a syringe or source of pressurized gas may be used to spray or eject the therapeutic agent from the catheter into the target site. However, such a delivery technique may result in an interrupted or unclear image or view of the target site using the imaging device of the endoscope to which the catheter is operatively coupled. Additionally, in some circumstances, the catheter may become clogged with the agent and/or other particulate, requiring the catheter to be removed and replaced during the procedure.

### SUMMARY

The present embodiments provide systems and methods suitable for delivering a therapeutic agent to a target site while i) maintaining suitable visibility of the target site and surrounding environment, and ii) enabling removal and replacement of system components in circumstances where the system does not function properly.

In one aspect, a system for delivering an agent including a plurality of particles to a target site includes a catheter having a first wall defining an inner lumen for delivery of the agent to the target site, and a second wall surrounding the first wall to define an outer lumen therebetween for delivery of a fluid to the target site. The catheter may have a distal end terminating with a primary opening of the inner lumen and a secondary opening of the outer lumen. A hub associated with a proximal end of the catheter includes a first inlet for receiving a supply of the agent and a second inlet for receiving a supply of the fluid, wherein the first inlet is in fluid communication with the inner lumen, and the second inlet is in fluid communication with the outer lumen. The hub may be fixed to the proximal end of the catheter.

The first inlet may include a male or female threaded connector. The second inlet may include a male or female threaded connector. The first inlet may include one of a male or female threaded connector, and the second inlet may include the other of the male or female threaded connector.

A first lumen may extend through the hub from the first inlet to a first outlet in fluid communication with the inner lumen of the catheter, with the first lumen being axially aligned with the inner lumen.

A second lumen may extend through the hub from the second inlet to a second outlet in fluid communication with the outer lumen of the catheter, with the second lumen at least partially surrounding the first lumen at the second outlet.

The hub comprises a connection interface, wherein the first inlet and the second inlet are formed on the connection interface. A gasket may surround the first inlet, while the second inlet may be positioned outside the gasket.

The hub may include a first connection interface and a second connection interface separate from the first connection interface, with the first inlet being positioned on the first connection interface, and with the second inlet being positioned on the second connection interface.

The system may include any one or more of the foregoing features.

In another aspect, a system for delivering an agent including a plurality of particles to a target site includes a catheter having a first wall defining an inner lumen for delivery of the agent to the target site, and a second wall surrounding the first wall to define an outer lumen therebetween for delivery of a fluid to the target site. The catheter may have a distal end terminating with a primary opening of the inner lumen and a secondary opening of the outer lumen. A hub associated with a proximal end of the catheter includes a first connection interface having a first inlet for receiving a supply of the agent, and a second connection interface having a second inlet for receiving a supply of the fluid, wherein the first connection interface is separate from the second connection interface, the first inlet is in fluid communication with the inner lumen, and the second inlet is in fluid communication with the outer lumen.

The first inlet may include a male or female threaded connector. The second inlet may include a male or female threaded connector. The first inlet may include one of a male or female threaded connector, and the second inlet may include the other of the male or female threaded connector.

A first lumen may extend through the hub from the first inlet to a first outlet in fluid communication with the inner lumen of the catheter, with the first lumen being axially aligned with the inner lumen.

A second lumen may extend through the hub from the second inlet to a second outlet in fluid communication with the outer lumen of the catheter, with the second lumen at least partially surrounding the first lumen at the second outlet.

The system may include any one or more of the foregoing features.

In yet another aspect, a system for delivering an agent including a plurality of particles to a target site including a catheter having a first wall defining an inner lumen for delivery of the agent to the target site, and a second wall surrounding the first wall to define an outer lumen therebetween for delivery of a fluid to the target site. The catheter may have a distal end terminating with a primary opening of the inner lumen and a secondary opening of the outer lumen. A hub associated with a proximal end of the catheter includes a connection interface having a first inlet for receiving a supply of the agent and a second inlet for receiving a supply of the fluid. The first inlet may be in fluid communication with the inner lumen, while the second inlet may be in fluid communication with the outer lumen. A gasket may surround the first inlet, and the second inlet may be positioned outside the gasket. The connection interface may include a male or female threaded connector.

A first lumen may extend through the hub from the first inlet to a first outlet in fluid communication with the inner lumen of the catheter, with the first lumen being axially aligned with the inner lumen. A second lumen may extend through the hub from the second inlet to a second outlet in fluid communication with the outer lumen of the catheter, with the second lumen at least partially surrounding the first lumen at the second outlet.

The system may include any one or more of the foregoing features.

In a further aspect, a system for delivering an agent including a plurality of particles to a target site includes a catheter having a first wall defining an inner lumen for delivery of the agent to the target site, and a second wall surrounding the first wall to define an outer lumen therebetween for delivery of a fluid to the target site. The catheter may include a distal end terminating with a primary opening of the inner lumen and a secondary opening of the outer lumen. A hub associated with a proximal end of the catheter may include a first inlet for receiving a supply of the agent and a second inlet for receiving a supply of the fluid, wherein the first inlet is in fluid communication with the inner lumen, and the second inlet is in fluid communication with the outer lumen. A handle may house a supply of the agent and a supply of the fluid. The catheter and the hub may be removably attached to the handle.

The hub may be fixed to the proximal end of the catheter.

The first inlet may include a male or female threaded connector, and the handle may include a corresponding mating male or female threaded connector. The second inlet may include a male or female threaded connector, and the handle may include a corresponding mating male or female threaded connector.

The first inlet may include one of a male or female threaded connector, and the second inlet include the other of the male or female threaded connector, with the handle including corresponding mating male or female threaded connectors.

A first lumen may extend through the hub from the first inlet to a first outlet in fluid communication with the inner lumen of the catheter, with the first lumen receiving a supply of the agent form the handle, and being axially aligned with the inner lumen. A second lumen may extend through the hub from the second inlet to a second outlet in fluid communication with the outer lumen of the catheter, with the second lumen receiving a supply of the fluid form the handle, the second lumen at least partially surrounding the first lumen at the second outlet.

The hub may include a connection interface for connection with the handle, wherein the first inlet and the second inlet are formed on the connection interface. A gasket may surround the first inlet, with the second inlet being positioned outside the gasket.

The hub may include a first connection interface for connection with the handle, and a second connection interface for connection with the handle, separate from the first connection interface, the first inlet being positioned on the first connection interface, and the second inlet being positioned on the second connection interface.

The system may include any one or more of the foregoing features.

In yet another aspect, a system for delivering an agent including a plurality of particles to a target site includes a catheter having a first wall defining an inner lumen for delivery of the agent to the target site, and a second wall surrounding the first wall to define an outer lumen therebetween for delivery of a fluid to the target site. The catheter may include a distal end terminating with a primary opening of the inner lumen and a secondary opening of the outer lumen. A hub associated with a proximal end of the catheter may include a first connection interface having a first inlet for receiving a supply of the agent, and a second connection interface having a second inlet for receiving a supply of the fluid. A handle may house a supply of the agent and a supply of the fluid. The catheter and the hub may be removably attached to the handle. The first connection interface may be separate from the second connection interface, with the first inlet in fluid communication with the inner lumen, and the second inlet in fluid communication with the outer lumen.

The first inlet may include a male or female threaded connector, and the handle may include a corresponding mating male or female threaded connector. The second inlet may include a male or female threaded connector, and the handle may include a corresponding mating male or female threaded connector. The first inlet may include one of a male or female threaded connector, and the second inlet may include the other of the male or female threaded connector, with the handle including corresponding mating male or female threaded connectors.

A first lumen may extend through the hub from the first inlet to a first outlet in fluid communication with the inner lumen of the catheter, with the first lumen receiving a supply of the agent form the handle, and being axially aligned with the inner lumen. A second lumen may extend through the hub from the second inlet to a second outlet in fluid communication with the outer lumen of the catheter, the second lumen receiving a supply of the fluid form the handle, and the second lumen at least partially surrounding the first lumen at the second outlet.

The system may include any one or more of the foregoing features.

In yet a further aspect, a system for delivering an agent including a plurality of particles to a target site includes a catheter having a first wall defining an inner lumen for delivery of the agent to the target site, and a second wall surrounding the first wall to define an outer lumen therebetween for delivery of a fluid to the target site. The catheter may have a distal end terminating with a primary opening of the inner lumen and a secondary opening of the outer lumen. A hub associated with a proximal end of the catheter may include a connection interface having a first inlet for receiving a supply of the agent and a second inlet for receiving a supply of the fluid. A handle may house a supply of the agent and a supply of the fluid. The catheter and the hub may be removably attached to the handle. The first inlet may be in fluid communication with the inner lumen, and the second inlet may be in fluid communication with the outer lumen.

An inner gasket may surround the first inlet, with the second inlet being positioned outside the gasket. An outer gasket may surround the second inlet and the inner gasket. The inner gasket and the outer gasket may be positioned on the connection interface. Alternatively, the inner gasket and the outer gasket may be positioned on the handle.

The connection interface may include a male or female threaded connector, and the handle may include a corresponding mating male or female threaded connector.

A first lumen may extend through the hub from the first inlet to a first outlet in fluid communication with the inner lumen of the catheter, with the first lumen receiving a supply of the agent form the handle, and being axially aligned with the inner lumen. A second lumen may extend through the hub from the second inlet to a second outlet in fluid communication with the outer lumen of the catheter, with the second lumen receiving a supply of the fluid form the handle, the second lumen at least partially surrounding the first lumen at the second outlet.

The system may include any one or more of the foregoing features.

Other systems, methods, features and advantages of the described embodiments will be, or will become, apparent to one with skill in the art upon examination of the following figures and detailed description. It is intended that all such additional systems, methods, features and advantages be within the scope of the disclosure, and be encompassed by the following claims.

### BRIEF DESCRIPTION OF THE DRAWINGS

The embodiments can be better understood with reference to the following drawings and description. The components in the figures are not necessarily to scale, emphasis instead being placed upon illustrating the principles of the disclosure. Moreover, in the figures, like referenced numerals designate corresponding parts throughout the different views.
FIG. 1 is a perspective view of an example endoscope system according to certain embodiments;
FIG. 2 is a cross-sectional schematic view of a portion of an example catheter suitable for use with the endoscope system of FIG. 1 according to certain embodiments;
FIG. 3 is a perspective view of a portion of the endoscope system with the catheter in a distally extended position;
FIG. 4 is a representative image of a target site in which the catheter is in the extended position, and fluid flow through the secondary openings forms a fluid curtain over or near lens to prevent or limit accumulation of debris on or near lens and maintain suitable visibility of the target site through lens; and
FIG. 5 is a representative image of the target site shown in FIG. 5 without the catheter providing the fluid curtain.
FIG. 6 is a cross-sectional schematic view of an example hub suitable for use with a proximal end of the catheter of FIG. 2;
FIG. 7 is a side view of an example handle suitable for use with the catheter and hub of FIGS. 2 and 6;
FIG. 8 is a cross-sectional schematic view of another example hub and a portion of a handle suitable for use with a proximal end of the catheter of FIG. 2;
FIG. 9 is a cross-sectional schematic view of another example hub and a connection interface of a handle (not shown) suitable for use with a proximal end of the catheter of FIG. 2; and,
FIG. 10 is a cross-sectional view of the intersection of the hub and connection interface of FIG. 9, taken along the dashed line in FIG. 9.

### DETAILED DESCRIPTION

In the present application, the term "proximal" refers to a direction that is generally towards a physician during a medical procedure, while the term "distal" refers to a direction that is generally towards a target site within a patient's anatomy during a medical procedure. As used herein to describe example embodiments, the term "fluid" may refer to a gas or a liquid.

In example embodiments as described herein, a system, such as an endoscope system, suitable for delivering an agent, such as a therapeutic agent, to a target site within a patient's anatomy. The system includes an endoscope having a proximal end and a distal end opposite the proximal end. The endoscope includes an imaging device extending through at least a portion of the endoscope between the proximal end and the distal end. The imaging device includes a lens positioned at a surface of the distal end of the endoscope. The lens is operatively coupled, such as in signal communication, to an external imaging system which receives one or more signals transmitted by the lens indicative of images captured by the lens of the target site and surrounding environment. The external imaging system includes one or more processors for processing the received signals and displaying on a display one or more images of the target site captured by the lens.

An accessory channel extends through at least a portion of the endoscope between the proximal end and the distal end. The accessory channel terminates at the surface of the distal end of the endoscope to form an opening at the surface of the distal end of the endoscope. In example embodiments, a catheter is movably positioned within the accessory channel. The catheter has a first wall defining an inner lumen sized for delivery of the agent to the target site, and a second wall surrounding the first wall to define an outer lumen therebetween for delivery of a fluid to the target site. In some embodiments, the first wall and the second all are coaxial, such that the inner lumen and the outer lumen are coaxial. The catheter has a distal end terminating with a primary opening, e.g., an outlet, of the inner lumen, and a secondary opening, e.g., an outlet, of the outer lumen. An opposing proximal end terminates with an inner lumen inlet opening, and an outer lumen inlet opening. A longitudinal axis extends between the distal end and the proximal end. In some embodiments, the second wall has one or more secondary openings formed at the distal end of the catheter such that fluid, e.g., gas such as carbon dioxide, is allowed to flow through the secondary opening or secondary openings at the distal end of the catheter to create a steady flow of fluid at an angle with respect to the longitudinal axis.

During operation, a pressurized fluid, e.g., a gas such as carbon dioxide, is advanced through the inner lumen to carry the agent particles to the target site, as well as through the outer lumen to supply the steady flow of fluid through the secondary opening or secondary openings. With the catheter in an extended position, i.e., extended distally from the accessory channel, the agent particles exit the inner lumen through the primary opening, while the fluid also exits the catheter through the secondary opening or secondary openings to create a steady fluid flow forming a fluid curtain over or near the lens to prevent or limit agent particles from obscuring or interrupting an image or view of the target site through the imaging device of the endoscope. In addition or alternatively, the fluid curtain may also be used to protect other structures, such as anatomical structures or devices, other than or in addition to the image device. As used herein to describe example embodiments, the term "fluid" may refer to a gas or a liquid.

In certain example embodiments, the catheter is movable within the accessory channel in a distal direction and an opposing proximal direction. With the catheter in an extended position, the catheter extends distally from the surface at the distal end of the endoscope such that the primary opening or primary openings are external to the accessory channel. One or more indicator marks may be located at the distal end of the catheter that are visible with the catheter in an extended position to indicate to the user that the catheter is extended distally a distance from the surface at the distal end of the endoscope. With the catheter in the extended position, the agent particles exit the inner lumen through the primary opening or primary openings, while the fluid exits through the secondary opening(s) in the outer lumen to create a steady fluid flow forming a fluid curtain over or near the lens to prevent or limit particles from obscuring or interrupting an image or view of the target site through the imaging device of the endoscope. In addition or alternatively, the fluid curtain may also be used to protect other structures, such as anatomical structures or devices, other than or in addition to the image device.

In some embodiments, the secondary opening at the distal end of the catheter is an annular slit formed in the second wall. In an alternative embodiment, the secondary opening at the distal end of the catheter is a plurality of discontinuous annular slit sections formed in the second wall.

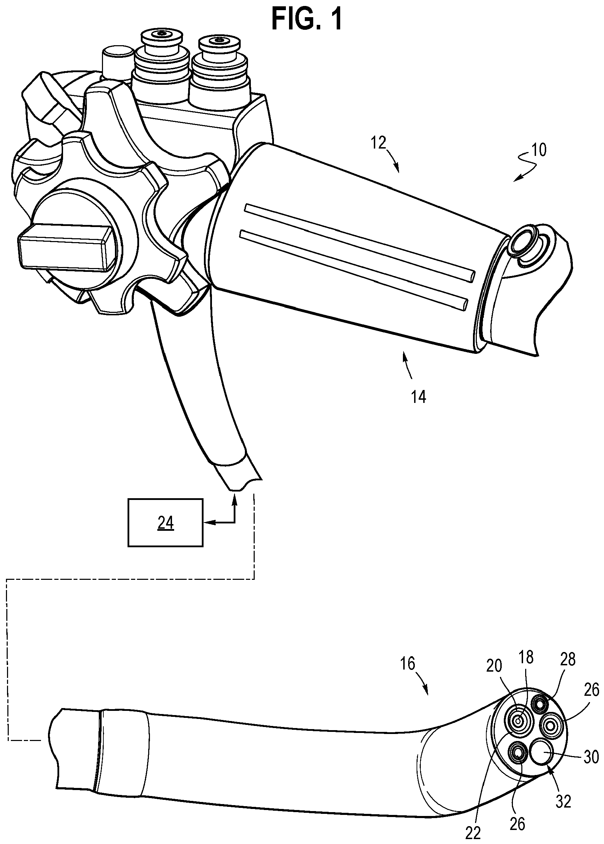

Referring now to FIGS. 1-5, in example embodiments, a system 10, such as an endoscope system, suitable for delivering an agent to a target site within a patient's anatomy includes an endoscope 12, shown in FIG. 1. Generally, endoscope 12 has a proximal end 14 and a distal end 16 opposite proximal end 14. An imaging device 18 extends through at least a portion of endoscope 12 between proximal end 14 and distal end 16. Imaging device 18 including a lens 20 positioned at a surface 22 of distal end 16. Lens 20 is operatively coupled to, e.g., in signal communication with, an external imaging system 24 and is configured to capture images of the target site and transmit signals indicative of the captured images to imaging system 24. As shown in FIG. 1, for example, endoscope 12 also includes one or more light sources 26, such as light-emitting diodes (LEDs), and a water source 28 configured to inject water into the target site.

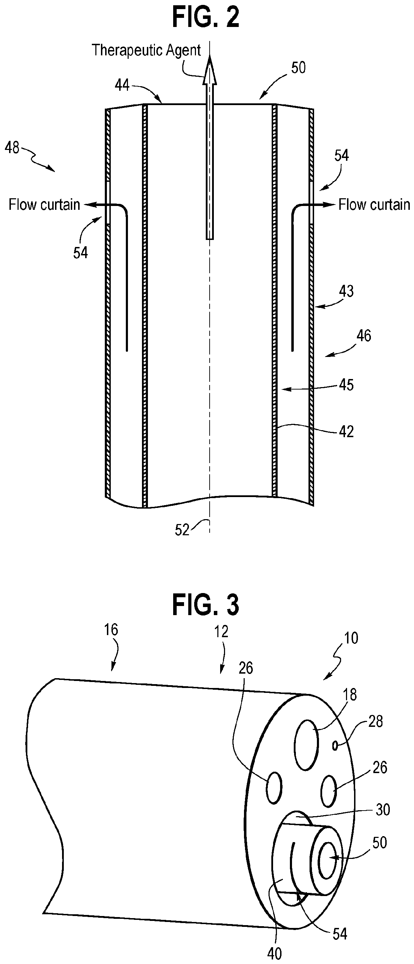

In example embodiments, endoscope 12 includes an accessory channel 30 extending through at least a portion of endoscope 12 between proximal end 14 and distal end 16. Accessory channel 30 terminates at surface 22 at distal end 16 forming an opening 32 at surface 22. Referring further to FIG. 2, system 10 also includes a catheter 40 that is movably positioned within accessory channel 30. Catheter 40 may include one or more suitable tube members for delivering the agent to a target site. For example, catheter 40 may comprise a flexible, tubular member that may be formed from one or more semi-rigid polymers including, without limitation, polyurethane, polyethylene, tetrafluoroethylene, polytetrafluoroethylene, fluorinated ethylene propylene, nylon, PEBAX or the like.

In operation, a distal end of catheter 40 may be positioned in relatively close proximity to the target site. Catheter 40 may be advanced to the target site using a flexible endoscopic technique, an open technique, a laparoscopic technique, an intraluminal technique, using a gastroenterology technique through the mouth, colon, or using any other suitable technique. Catheter 40 may comprise one or more markers configured to be visualized under fluoroscopy or other imaging techniques to facilitate location of the distal end of catheter 40. As described herein, catheter 40 may be advanced through a working lumen, e.g., an accessory channel 30, of an endoscope 12. When the catheter 90 is positioned at the desired target site, a pressure source urges the pressurized fluid to flow through catheter 40, thereby delivering the agent to the target site at a desired pressure.

Catheter 40 includes a first wall 42 defining an inner lumen 44 sized for delivery of the agent, such as a therapeutic agent, to the target site, and a second wall 43 surrounding the first wall 42 to define an outer lumen 45 therebetween, sized for delivery of the fluid to the target site. In some embodiments, first wall 42 and second wall 43 are coaxial, such that inner lumen 44 and outer lumen 45 are coaxial.

Catheter 40 has a proximal end 46 and an opposing distal end 48 terminating with a primary opening 50 into the inner lumen 44, and one or more secondary openings 54 into the outer lumen 45. A longitudinal axis 52 extends between proximal end 46 and distal end 48 of catheter 40. As discussed herein, the proximal end 46 of the catheter 40 terminates with a hub 56 having one or more connection interfaces for removable connection of the catheter to a source of the therapeutic agent and/or a source of pressurized gas, enabling the catheter to be removed and replaced during a procedure.

Secondary opening(s) 54 may have any suitable shape and/or configuration including, without limitation, an orifice, a slit, and/or an aperture formed in the second wall 43 at distal end 48 of catheter 40. Each secondary opening 54 is configured to have an area or dimensions such that fluid, e.g., gas such as carbon dioxide or a liquid, is allowed to flow through each secondary opening 54 to create a steady flow of fluid at an angle with respect to longitudinal axis 52. In example embodiments, an axis of at least one secondary opening 54, defined by the direction of flow through the opening, is positioned at an angle with respect to longitudinal axis 52 of 30° to 150°. In certain example embodiments, secondary opening 54 is positioned such that the fluid, e.g., gas, flows tangential to longitudinal axis 52.

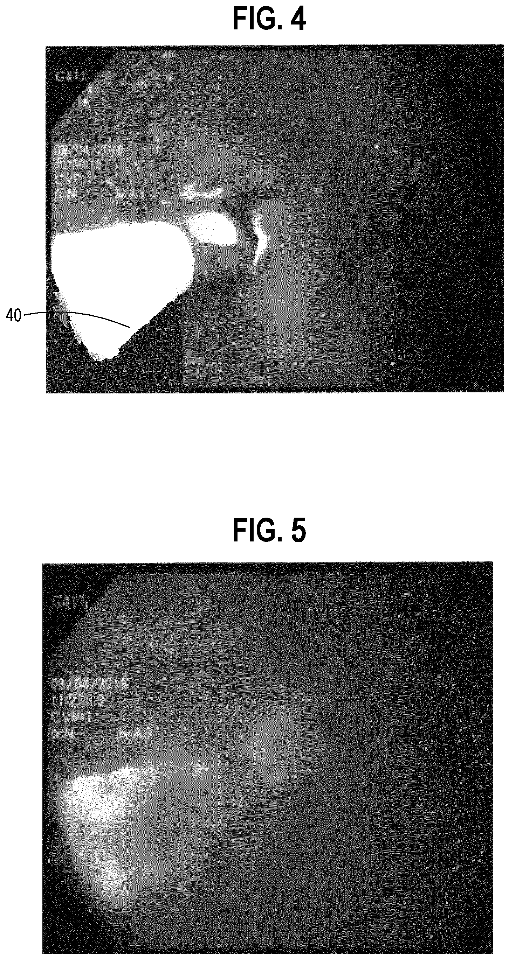

As shown, for example, in FIG. 3, catheter 40 is movable within accessory channel 30 in a distal direction along longitudinal axis 52 and an opposite proximal direction along longitudinal axis 52. With catheter 40 in an extended position shown in FIG. 4, catheter 40 extends distally from surface 22 such that each secondary opening 54 is external to accessory channel 30. Catheter 40 may include one or more indicator marks at distal end 48 to indicate to the user that catheter 40 is extended distally a distance from surface 22 at distal end 16 of endoscope 12. With catheter 40 in the extended position, the fluid flow through the secondary openings 54 forms a fluid curtain over or near lens 20 to prevent or limit accumulation of debris on or near lens 20, to maintain suitable visibility of the target site through lens 20.

Referring now to FIG. 2, in an example embodiment, secondary opening(s) 54 may include a plurality of discontinuous slit sections positioned about the circumference of second wall 43 to form a discontinuous annular slit. In certain example embodiments, with catheter 40 in an extend position, annular slit 54 is positioned not greater than 10.0 cm from distal end 48 of catheter 40 and, in particular embodiments, annular slit 54 is positioned not greater than 2.0 cm from distal end 48 of catheter 40. In a particular embodiment, an axis of annular slit 54 is positioned at an angle with respect to longitudinal axis 52 of 30 degrees to 150 degrees.

In one embodiment, the catheter is suitable for delivering an agent, such as a therapeutic agent, to a target site. The catheter has an inner lumen sized for delivery of the agent to the target site, and an outer lumen sized for delivery of the fluid to the target site. The catheter has a distal end, an opposing proximal end, and a longitudinal axis extending between the distal end and the proximal end. A plurality of secondary openings are formed through a wall of the outer lumen of the catheter at the distal end. Each secondary opening of the plurality of secondary openings allows the fluid, e.g., gas, to flow through the plurality of secondary openings to create a steady flow of fluid at an angle with respect to the longitudinal axis, e.g., tangential to the distal end of the catheter. The system includes an endoscope having a proximal end and a distal end opposite the proximal end. The endoscope includes an imaging device that extends through at least a portion of the endoscope between the proximal end and the distal end. The imaging device includes a lens positioned at a surface of the distal end of the endoscope. An accessory channel extends through at least a portion of the endoscope between the proximal end and the distal end of the endoscope. The accessory channel terminates at the surface of the distal end of the endoscope to form an opening at the surface of the distal end of the endoscope. The catheter is movable within the accessory channel in a distal direction and an opposing proximal direction. With the catheter in an extended position, the catheter extends distally from the surface of the distal end of the endoscope such that the secondary openings are external to the accessory channel. In a particular embodiment, an indicator mark at the distal end of the catheter indicates to the user that the catheter is extended distally a distance from the surface of the distal end. With the catheter in the extended position, the fluid flow through the secondary openings forms a fluid curtain over or near lens to prevent or limit accumulation of debris on or near lens and maintain suitable visibility of the target site through lens.

FIG. 4 is representative of an image of a target site in which catheter 40 is in an extended position and provides the fluid flow through secondary opening(s) 54 to form a fluid curtain over or near lens 20 to prevent or limit accumulation of debris on or near lens 20 and maintain suitable visibility of the target site through lens 20. Conversely, FIG. 5 is representative of an image of the target site shown in FIG. 4 without catheter 40 in the extend position and providing the fluid flow forming the fluid curtain over or near lens 20.

System 10 may be used to deliver the agent in a wide range of procedures and the agent, e.g., a therapeutic agent, may be chosen to perform a desired function upon ejection from distal end 48 of catheter 40. For example, the provision of the therapeutic agent may be used for providing hemostasis, closing perforations, performing lithotripsy, treating tumors and cancers, treat renal dialysis fistulae stenosis, or vascular graft stenosis. The agent can be delivered during procedures such as coronary artery angioplasty, renal artery angioplasty and carotid artery surgery, or may be used generally for treating various other cardiovascular, respiratory, gastroenterology or other conditions. The above-mentioned systems also may be used in transvaginal, umbilical, nasal, and bronchial/lung related applications.

For example, if used for purposes of hemostasis, thrombin, epinephrine, or a sclerosant may be provided to reduce localized bleeding. Similarly, if used for closing a perforation, a fibrin sealant may be delivered to a localized lesion. In addition to the hemostatic properties of the agent, it should be noted that the relatively high pressure of the fluid and the agent, by itself, may act as a mechanical tamponade by providing a compressive force, thereby reducing the time needed to achieve hemostasis.

The agent may be selected to perform one or more desired biological functions, for example, promoting the ingrowth of tissue from the interior wall of a body vessel, or alternatively, to mitigate or prevent undesired conditions in the vessel wall, such as restenosis. Many other types of agents may be used in conjunction with system 10.

The agent may be delivered in any suitable form. For example, the agent may comprise a powder, liquid, gel, aerosol, other substances or combinations thereof. Advantageously, the pressure source may facilitate delivery of the agent in any one of these forms. The agent employed also may include an antithrombogenic bioactive agent, e.g., any bioactive agent that inhibits or prevents thrombus formation within a body vessel. Types of antithrombotic bioactive agents include anticoagulants, antiplatelets, and fibrinolytics. Anticoagulants are bioactive materials which act on any of the factors, cofactors, activated factors, or activated cofactors in the biochemical cascade and inhibit the synthesis of fibrin. Antiplatelet bioactive agents inhibit the adhesion, activation, and aggregation of platelets, which are key components of thrombi and play an important role in thrombosis. Fibrinolytic bioactive agents enhance the fibrinolytic cascade or otherwise aid in dissolution of a thrombus. Examples of antithrombotics include but are not limited to anticoagulants such as thrombin, Factor Xa, Factor VIIa and tissue factor inhibitors; antiplatelets such as glycoprotein IIb/IIIa, thromboxane A2, ADP-induced glycoprotein IIb/IIIa, and phosphodiesterase inhibitors; and fibrinolytics such as plasminogen activators, thrombin activatable fibrinolysis inhibitor (TAFI) inhibitors, and other enzymes which cleave fibrin.

Additionally, or alternatively, the agent may include thrombolytic agents used to dissolve blood clots that may adversely affect blood flow in body vessels. A thrombolytic agent is any therapeutic agent that either digests fibrin fibers directly or activates the natural mechanisms for doing so. Examples of commercial thrombolytics, with the corresponding active agent in parenthesis, include, but are not limited to, Abbokinase (urokinase), Abbokinase Open-Cath (urokinase), Activase (alteplase, recombinant), Eminase (anitstreplase), Retavase (reteplase, recombinant), and Streptase (streptokinase). Other commonly used names are anisoylated plasminogen-streptokinase activator complex; APSAC; tissue-type plasminogen activator (recombinant); t-PA; rt-PA. The therapeutic agent 38 may comprise coating-forming agents to protect or assist in healing of lesions and/or wounds. While a few exemplary agents have been described, it will be apparent that numerous other suitable agents, e.g., therapeutic agents, may be used in conjunction with system 10 and delivered through catheter 40.

Advantageously, system 10 permits localized delivery of a desired quantity of the agent at a desired, regulated pressure. Since distal end 48 of catheter 40 may be placed in relatively close proximity to a target site, system 10 provides significant advantages over agents delivered orally or through an IV system and may reduce accumulation of the agent in healthy tissues, thereby reducing side effects. Moreover, the delivery of the agent to the target site is performed in a relatively fast manner due to the relatively high pressure of the fluid, thereby providing a prompt delivery to the target site compared to previous devices.

Further, if an optional needle is employed at distal end 48 of catheter 40, system 10 advantageously may be used to both perforate tissue at or near a target site, then deliver the agent at a desired pressure in the manner described above. For example, the needle may comprise an endoscopic ultrasound (EUS) needle. Accordingly, in one exemplary technique, a sharpened tip of the needle may be capable of puncturing through an organ or a gastrointestinal wall or tissue, so that the agent may be delivered at a predetermined pressure in various bodily locations that may be otherwise difficult to access. One or more delivery vehicles, such as an endoscope or sheath, may be employed to deliver catheter 40 to a target site, particularly if distal end 48 of catheter 40 includes the optional needle.

In example embodiments, the agent has a specific range of properties that make it suitable for delivery through catheter 40, particularly when catheter 40 is sized for delivery through a lumen of an endoscope. In particular, the mass of an individual particle of the agent should be within a specific range. If a particle of the agent is too heavy, it will require too much pressure to travel the length of catheter 40 and may result in clogging of catheter 40, requiring the catheter 40 to be removed and replaced during the procedure. If the particle is too light, it will aerosolize within the patient's body, e.g., in the gastrointestinal space, instead of being propelled to a target site. In addition to mass of an individual particle of the agent, the size of the particle is important for ensuring proper delivery through catheter 40. If the agent particle is too large in size, then it will be prone to clogging within catheter 40, requiring the catheter 40 to be removed and replaced during the procedure.. If the particle is too small, it may have a higher likelihood of being aerosolized instead of being propelled to the target site.

Similarly, with regard to dimensions of catheter 40, when used in endoscopic applications, it is clinically important to size catheter 40 to be small enough to fit through a working lumen of the endoscope, e.g., the accessory channel, yet be large enough to substantially avoid clogging when the agent is advanced through catheter 40.

With regard to pressure, as noted above, the pressure source may include a pressurized fluid cartridge of a selected gas or liquid, such as carbon dioxide, nitrogen, or any other suitable gas or liquid that may be compatible with the human body. The pressurized fluid cartridge may contain the gas or liquid at a relatively high, first predetermined pressure, for example, around 1,800 pounds per square inch (psi) inside of the cartridge. The pressure source may be in a solid (dry ice), liquid or gas state. As further noted above, the fluid may flow from the pressure source through a pressure regulator, such as a regulator valve having a pressure outlet, which may reduce the pressure to a lower, second predetermined pressure (referred to here as a "delivery system pressure"). In one embodiment, it has been found beneficial to have a delivery system pressure in the range of 0.01 psi to 100 psi, and, more particularly, in the range of about 0.5 psi to about 75 psi. Such ranges may have criticality in terms of providing appropriate force to propel the agent through catheter 40, while significantly reducing the likelihood of clogging of catheter 40 during delivery, and therefore properly deliver the agent to a target site in the correct dose.

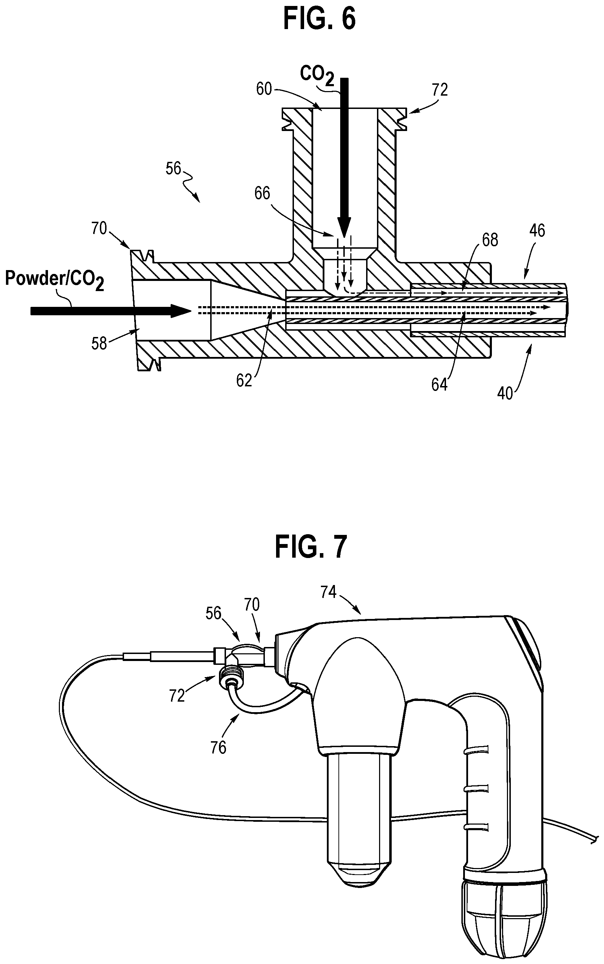

In circumstances where catheter 40 becomes clogged, or otherwise does not function properly, catheter 40 must be removed from the system and replaced with a new catheter 40. In that regard, as shown in FIGS. 6-10, the proximal end 46 of the catheter 40 terminates with a hub 56 having one or more connection interfaces for removable connection of the catheter to a source of the therapeutic agent, and/or a source of pressurized gas, enabling the catheter 40 to be removed and replaced with a new catheter 40 during a procedure. The hub 56 may be fixed to the proximal end 46 of the catheter 40 by any suitable means. Alternatively, the hub 56 may be removably attached to the proximal end 46 of the catheter 40.

Referring to FIG. 6, in one embodiment, hub 56 includes a first connection interface 70 having a first inlet 58 for receiving a supply of the agent (or an agent and a fluid), and a second connection interface 72, separate from the first connection interface 70, having a second inlet 60 for receiving a supply of the fluid. The first inlet 58 is in fluid communication with the inner lumen 44 of the catheter 40, while the second inlet 60 is in fluid communication with the outer lumen 45 of the catheter 40. More specifically, a first lumen 62 extends through the hub 56 from the first inlet 58 to a first outlet 64 in fluid communication with the inner lumen 44 of the catheter 40, while a second lumen 66 extends through the hub 56 from the second inlet 60 to a second outlet 68 in fluid communication with the outer lumen 45 of the catheter 40.

As illustrated in FIG. 6, the agent may be in the form of a powder that is transferred through the inner lumen 44 to the distal end 48 of the catheter 40 via a supply of pressurized carbon dioxide, while the fluid may be a gas that is transferred through the outer lumen 45 to the distal end 48 of the catheter 40 via the same or a different supply of pressurized carbon dioxide.

In some embodiments, the first lumen 62 is axially aligned with the inner lumen 44 of the catheter, such that the flow of the agent from the supply through the hub 56 and into the inner lumen 44 is substantially straight, thereby reducing the likelihood that the agent clogs in the hub 56, as compared to a lumen that includes one or more turns or bends. In alternative embodiments, the first lumen 62 may include one or more turns or bends. The first lumen 62 may also include one or more reductions in cross-sectional area in order to increase the velocity of the agent flowing through the hub 56. Likewise, although the second lumen 66 is shown as having one 90-degree turn between the second inlet 60 and the second outlet 68, it should be appreciated that the second inlet 60 and the second lumen 66 may be oriented at any number of angles relative to the first inlet 58 and/or the first lumen 62.

In some embodiments, the first connection interface 70 and/or the first inlet 58 may comprise a male or a female threaded connection for removably connecting the hub 56 to structure housing a supply of the agent and a supply of pressurized fluid. Likewise the second connection interface 72 and/or the second inlet 60 may comprise a male or female threaded connection for removably connecting the hub 56 to a structure housing a supply of pressurized fluid. In some embodiments, the male or female threaded connection is a male or female luer component.

In one embodiment, as shown in FIG. 7, the hub 56 may be removably attached to a handle 74 that houses the supply of the agent and a source of pressurized fluid. The hub 56 may be removably attached to the handle 74 via the first connection interface 70 and a corresponding mating male or female threaded connector on the handle 74. The second connection interface 72 may be connected in fluid communication with a supply of pressurized fluid within the handle 74 via tubing 76 having a corresponding mating male or female threaded connector on an end of the tubing 76. In some embodiments, the handle 74 may have one or more actuation mechanisms to selectively start or stop supply of the agent and a fluid to the first connection interface 70 and/or the supply of the fluid to the second connection interface 72.

When used in a procedure, the embodiments of FIGS. 6-7 permit a user to remove and replace catheter 40, as necessary, with a minimal number of steps. For example, the user may disconnect the second connection interface 72 via rotation of that male or female threaded connector, followed by disconnection of the first connection interface 70 via rotation of that male or female connector. Then, the user may connect a new catheter 40 to the handle 74 by connecting the first connection interface 70 via rotation of that male or female connector, followed by connection of the second connection interface 72 via rotation of that male or female threaded connector. In some embodiments, the first connection interface 70 may have male and female threaded connectors unique to the first connection interface 70, while the second connection interface 72 may have male and female threaded connectors unique to the second connection interface 72, such that the first connection interface 70 may only be connected to the handle 74 at one location, while the second connection interface 72 may only be connected to the handle 74 at a separate location, thereby preventing improper connection of the hub 56 to the handle 74.

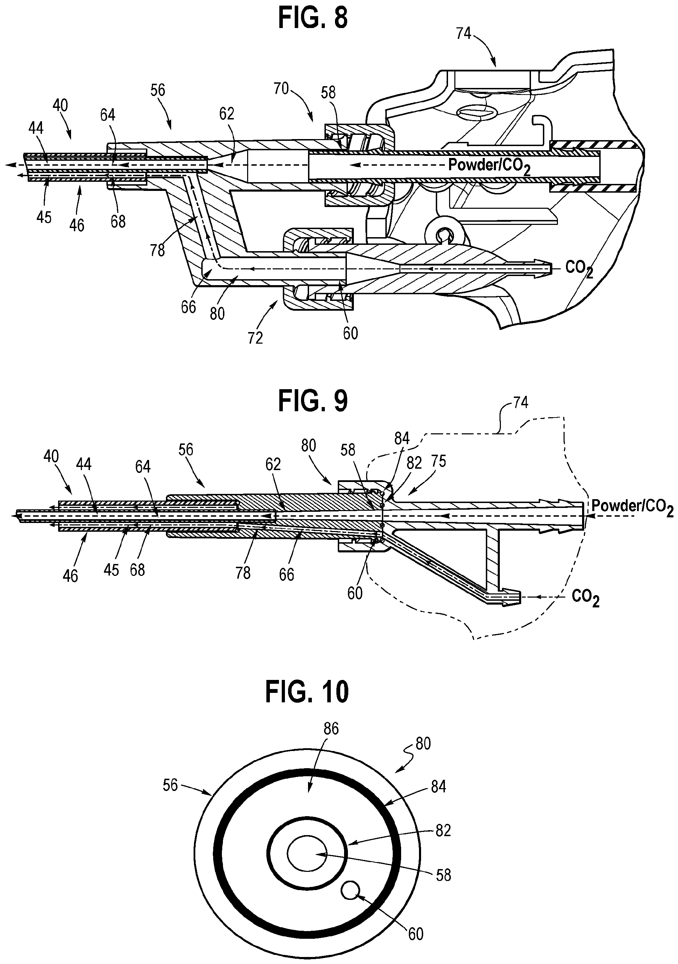

Referring to FIG. 8, in another embodiment, hub 56 includes a first connection interface 70 having a first inlet 58 for receiving a supply of the agent (or an agent and a fluid), and a second connection interface 72, separate from the first connection interface 70, having a second inlet 60 for receiving a supply of the fluid. The first inlet 58 is in fluid communication with the inner lumen 44 of the catheter 40, while the second inlet 60 is in fluid communication with the outer lumen 45 of the catheter 40. More specifically, a first lumen 62 extends through the hub 56 from the first inlet 58 to a first outlet 64 in fluid communication with the inner lumen 44 of the catheter 40, while a second lumen 66 extends through the hub 56 from the second inlet 60 to a second outlet 68 in fluid communication with the outer lumen 45 of the catheter 40.

As illustrated in FIG. 8, the agent may be in the form of a powder that is transferred through the inner lumen 44 to the distal end 48 of the catheter 40 via a supply of pressurized carbon dioxide, while the fluid may be a gas that is transferred through the outer lumen 45 to the distal end 48 of the catheter 40 via the same or a different supply of pressurized carbon dioxide.

In some embodiments, the first lumen 62 is axially aligned with the inner lumen 44 of the catheter, such that the flow of the agent from the supply through the hub 56 and into the inner lumen 44 is substantially straight, thereby reducing the likelihood that the agent clogs in the hub 56, as compared to a lumen that includes one or more turns or bends. In alternative embodiments, the first lumen 62 may include one or more turns or bends. The first lumen 62 may also include one or more reductions in cross-sectional area in order to increase the velocity of the agent flowing through the hub 56.

In the embodiment of FIG. 8, the second lumen 66 comprises an angled portion 78, extending at an angle relative to the first lumen 62 toward a handle 74, and a parallel portion 80 extending parallel to the first lumen 62 from the angled portion 78 to the handle 74. Although the angled portion 78 is shown as extending at an approximately 70-degree angle relative to the first lumen 62, other angles are envisioned, including from 0-degrees to 90-degrees. Additionally, the second lumen 66 may comprise one or more angled portions, and one or more parallel portions.

In some embodiments, the first connection interface 70 and/or the first inlet 58 may comprise a male or a female threaded connector for removably connecting the hub 56 to structure housing a supply of the agent and a supply of pressurized fluid. Likewise the second connection interface 72 and/or the second inlet 60 may comprise a male or female threaded connector for removably connecting the hub 56 to a structure housing a supply of pressurized fluid. In some embodiments, the male or female threaded connector is a male or female luer component.

In some embodiments, the hub 56 may be removably attached to a handle 74 that houses a supply of the agent and a source of pressurized fluid. The hub 56 may be removably attached to the handle 74 via the first connection interface 70 and a corresponding mating male or female threaded connector on the handle 74. The second connection interface 72 may be removably attached to the handle 74 via the second connection interface 72 and a corresponding mating male or female threaded connector on the handle 74. In some embodiments, the handle 74 may have one or more actuation mechanisms to selectively start or stop supply of the agent and a fluid to the first connection interface 70 and/or the supply of the fluid to the second connection interface 72.

In the embodiment of FIG. 8, the first connection interface 70 and/or the first inlet 58 comprises a male threaded connector for removably connecting the first connection interface 70 with a corresponding mating female threaded connector on the handle 74, while the second connection interface 72 and/or the second inlet 60 comprises a female threaded connector for removably connecting the second connection interface 72 with a corresponding male threaded connector on the handle 74. In some embodiments, the female threaded connector on the handle 74 may rotate relative to the handle 74, while the female threaded connector on the hub 56 may rotate relative to the hub. In this way, the first connection interface 70 and the second connection interface 72 may only be removably connected to the handle 74 in the orientation illustrated in FIG. 8, thereby preventing improper connection of the hub 56 to the handle 74 (i.e., by reversing the orientation of the first connection interface 70 and the second connection interface 72).

When used in a procedure, the embodiment of FIG. 8 permits a user to remove and replace catheter 40, as necessary, with a minimal number of steps. For example, the user may disconnect the first connection interface 70 via rotation of the female threaded connector associated with the handle 74, followed by disconnection of the second connection interface 72 via rotation of the female threaded connector associated with the hub 56. Then, the user may connect a new catheter 40 to the handle 74 by connecting the first connection interface 70 via rotation of the female threaded connector associated with the handle 74, followed by connection of the second connection interface 72 via rotation of the female threaded connector associated with the hub 56.

Referring to FIG. 9, in another embodiment, hub 56 includes a single connection interface 80 having both a first inlet 58 for receiving a supply of the agent (or an agent and a fluid), and a second inlet 60 for receiving a supply of the fluid. The first inlet 58 is in fluid communication with the inner lumen 44 of the catheter 40, while the second inlet 60 is in fluid communication with the outer lumen 45 of the catheter 40. More specifically, a first lumen 62 extends through the hub 56 from the first inlet 58 to a first outlet 64 in fluid communication with the inner lumen 44 of the catheter 40, while a second lumen 66 extends through the hub 56 from the second inlet 60 to a second outlet 68 in fluid communication with the outer lumen 45 of the catheter 40.

As illustrated in FIG. 9, the agent may be in the form of a powder that is transferred through the inner lumen 44 to the distal end 48 of the catheter 40 via a supply of pressurized carbon dioxide, while the fluid may be a gas that is transferred through the outer lumen 45 to the distal end 48 of the catheter 40 via the same or a different supply of pressurized carbon dioxide.

In some embodiments, the first lumen 62 is axially aligned with the inner lumen 44 of the catheter, such that the flow of the agent from the supply through the hub 56 and into the inner lumen 44 is substantially straight, thereby reducing the likelihood that the agent clogs in the hub 56, as compared to a lumen that includes one or more turns or bends. In alternative embodiments, the first lumen 62 may include one or more turns or bends.

In the embodiment of FIG. 9, the second lumen 66 comprises an angled portion 78, extending at an angle relative to the first lumen 62 toward a connection interface 75 associated with a handle 74 (not shown). Although the angled portion 78 is shown as extending at an approximately 20-degree angle relative to the first lumen 62, other angles are envisioned, including from 0-degrees to 45-degrees. Additionally, the second lumen 66 may comprise one or more angled portions, and one or more parallel portions, where the second lumen 66 is parallel to the first lumen 62.

In some embodiments, the single connection interface 80 may comprise a male or a female threaded connector for removably connecting the hub 56 to structure housing a supply of the agent and a supply of pressurized fluid. In some embodiments, the male or female threaded connector is a male or female luer component.

In the embodiment of FIG. 9, the hub 56 may be removably attached to a connection interface 75 associated with a handle 74 (not shown) that houses the supply of the agent and a source of pressurized fluid. The hub 56 may be removably attached to the connection interface 75 of the handle 74 via the connection interface 80 on the hub 56, and a corresponding mating male or female threaded connector on the handle 74. In some embodiments, the handle 74 may have one or more actuation mechanisms to selectively start or stop supply of the agent and a fluid to the first inlet 58 and/or the supply of the fluid to the second inlet 60.

In some embodiments, as illustrated in FIGS. 9-10, the hub 56 includes an inner gasket 82 positioned on the connection interface 80 to surround the first inlet 58 (but not the second inlet 60), and an outer gasket 84 positioned on the connection interface 80 to surround the second inlet 60 and the inner gasket 82. When the connection interface 80 of the hub 56 is threaded on the connection interface 75 of the handle 74, an annular channel 86 is formed between the inner gasket 82 and the outer gasket 84, in the radial direction, and the connection interface 80 of the hub 56 and the connection interface 75 of the handle 74, in the axial direction. Thus, regardless of whether the hub 56, upon connection to the connection interface 75 of the handle 74, is rotated to a position where the second inlet 60 aligns with the supply of the fluid from the handle 72, as illustrated in FIG 9, the fluid may flow from the handle 74, through the annular channel 86, to the second inlet 60, then into the second lumen 66. In some embodiments the inner gasket 82 and the outer gasket 84 may be rubber rings. In some embodiments, the inner gasket 82 and/or the outer gasket 84 may positioned on the connection interface 75 of the handle 75, rather than the connection interface 80 of the hub 56.

When used in a procedure, the embodiments of FIGS. 9-10 permit a user to remove and replace catheter 40, as necessary, with a minimal number of steps. For example, the user may disconnect the single connection interface 80 via rotation of that male or female threaded connector. Then, the user may connect a new catheter 40 to the handle 74 by connecting the single connection interface 80 to the connection interface 75 via rotation of that male or female connector. This embodiment has the benefit of requiring a user to complete only a single connection in order to establish independent supplies of an agent (or an agent and a fluid), and separately, a fluid.

Although the subject matter has been described in language specific to structural features and/or methodological acts, it is to be understood that the subject matter defined in the appended claims is not necessarily limited to the specific features or acts described. Rather, the specific features and acts are disclosed as illustrative forms of implementing the claims.

One skilled in the art will realize that a virtually unlimited number of variations to the above descriptions are possible, and that the examples and the accompanying figures are merely to illustrate one or more examples of implementations.
The present disclosure also provides the embodiments set out in the following numbered clauses:
C1. A system for delivering an agent including a plurality of particles to a target site, the system comprising:
   a catheter having a first wall defining an inner lumen for delivery of the agent to the target site, and a second wall surrounding the first wall to define an outer lumen therebetween for delivery of a fluid to the target site, the catheter having a distal end terminating with a primary opening of the inner lumen and a secondary opening of the outer lumen; and,
   a hub associated with a proximal end of the catheter and comprising a first inlet for receiving a supply of the agent and a second inlet for receiving a supply of the fluid, wherein the first inlet is in fluid communication with the inner lumen, and the second inlet is in fluid communication with the outer lumen.
C2. The system of clause 1, wherein the hub is fixed to the proximal end of the catheter.
C3. The system of clause 1, wherein the first inlet comprises a male or female threaded connector.
C4. The system of clause 1, wherein the second inlet comprises a male or female threaded connector.
C5. The system of clause 1, wherein the first inlet comprises one of a male or female threaded connector, and the second inlet comprises the other of the male or female threaded connector.
C6. The system of clause 1, wherein a first lumen extends through the hub from the first inlet to a first outlet in fluid communication with the inner lumen of the catheter, the first lumen being axially aligned with the inner lumen.
C7. The system of clause 6, wherein a second lumen extends through the hub from the second inlet to a second outlet in fluid communication with the outer lumen of the catheter, the second lumen at least partially surrounding the first lumen at the second outlet.
C8. The system of clause 7, wherein the hub comprises a connection interface, and wherein the first inlet and the second inlet are formed on the connection interface.
C9. The system of clause 8, wherein a gasket surrounds the first inlet, and the second inlet is positioned outside the gasket.
CI0. The system of clause 1, wherein the hub comprises a first connection interface and a second connection interface separate from the first connection interface, the first inlet being positioned on the first connection interface, and the second inlet being positioned on the second connection interface.
C11. A system for delivering an agent including a plurality of particles to a target site, the system according to any one or more of clauses 1 to 10.
C12. A system for delivering an agent including a plurality of particles to a target site, the system comprising:
   a catheter having a first wall defining an inner lumen for delivery of the agent to the target site, and a second wall surrounding the first wall to define an outer lumen therebetween for delivery of a fluid to the target site, the catheter having a distal end terminating with a primary opening of the inner lumen and a secondary opening of the outer lumen;
   a hub associated with a proximal end of the catheter and comprising a first connection interface having a first inlet for receiving a supply of the agent, and a second connection interface having a second inlet for receiving a supply of the fluid;
   wherein the first connection interface is separate from the second connection interface; and,
   wherein the first inlet is in fluid communication with the inner lumen, and the second inlet is in fluid communication with the outer lumen.
C13. The system of clause 12, wherein the first inlet comprises a male or female threaded connector.
C14. The system of clause 12, wherein the second inlet comprises a male or female threaded connector.
C15. The system of clause 12, wherein the first inlet comprise one of a male or female threaded connector, and the second inlet comprises the other of the male or female threaded connector.
C16. The system of clause 12, wherein a first lumen extends through the hub from the first inlet to a first outlet in fluid communication with the inner lumen of the catheter, the first lumen being axially aligned with the inner lumen.
C17. The system of clause 16, wherein a second lumen extends through the hub from the second inlet to a second outlet in fluid communication with the outer lumen of the catheter, the second lumen at least partially surrounding the first lumen at the second outlet.
C18. A system for delivering an agent including a plurality of particles to a target site, the system according to any one or more of clauses 12 to 17.
C19. A system for delivering an agent including a plurality of particles to a target site, the system comprising:
   a catheter having a first wall defining an inner lumen for delivery of the agent to the target site, and a second wall surrounding the first wall to define an outer lumen therebetween for delivery of a fluid to the target site, the catheter having a distal end terminating with a primary opening of the inner lumen and a secondary opening of the outer lumen; and,
   a hub associated with a proximal end of the catheter and comprising a connection interface having a first inlet for receiving a supply of the agent and a second inlet for receiving a supply of the fluid;
   wherein the first inlet is in fluid communication with the inner lumen, and the second inlet is in fluid communication with the outer lumen.
C20. The system of clause 19, wherein a gasket surrounds the first inlet, and the second inlet is positioned outside the gasket.
C21. The system of clause 19, wherein the connection interface comprises a male or female threaded connector.
C22. The system of clause 19, wherein a first lumen extends through the hub from the first inlet to a first outlet in fluid communication with the inner lumen of the catheter, the first lumen being axially aligned with the inner lumen, and wherein a second lumen extends through the hub from the second inlet to a second outlet in fluid communication with the outer lumen of the catheter, the second lumen at least partially surrounding the first lumen at the second outlet.
C23. A system for delivering an agent including a plurality of particles to a target site, the system according to any one or more of clauses 19 to 22.
C24. A system for delivering an agent including a plurality of particles to a target site, the system comprising:
   a catheter having a first wall defining an inner lumen for delivery of the agent to the target site, and a second wall surrounding the first wall to define an outer lumen therebetween for delivery of a fluid to the target site, the catheter having a distal end terminating with a primary opening of the inner lumen and a secondary opening of the outer lumen;
   a hub associated with a proximal end of the catheter and comprising a first inlet for receiving a supply of the agent and a second inlet for receiving a supply of the fluid, wherein the first inlet is in fluid communication with the inner lumen, and the second inlet is in fluid communication with the outer lumen; and,
   a handle for housing a supply of the agent, and a supply of the fluid;
   wherein the catheter and the hub are removably attached to the handle.
C25. The system of clause 24, wherein the hub is fixed to the proximal end of the catheter.
C26. The system of clause 24, wherein the first inlet comprises a male or female threaded connector, and the handle comprises a corresponding mating male or female threaded connector.
C27. The system of clause 24, wherein the second inlet comprises a male or female threaded connector, and the handle comprising a corresponding mating male or female threaded connector.
C28. The system of clause 24, wherein the first inlet comprises one of a male or female threaded connector, and the second inlet comprises the other of the male or female threaded connector, the handle comprising corresponding mating male or female threaded connectors.
C29. The system of clause 24, wherein a first lumen extends through the hub from the first inlet to a first outlet in fluid communication with the inner lumen of the catheter, the first lumen receiving a supply of the agent form the handle and being axially aligned with the inner lumen.
C30. The system of clause 29, wherein a second lumen extends through the hub from the second inlet to a second outlet in fluid communication with the outer lumen of the catheter, the second lumen receiving a supply of the fluid form the handle, the second lumen at least partially surrounding the first lumen at the second outlet.
C31. The system of clause 30, wherein the hub comprises a connection interface for connection with the handle, and wherein the first inlet and the second inlet are formed on the connection interface.
C32. The system of clause 31, wherein a gasket surrounds the first inlet, and the second inlet is positioned outside the gasket.
C33. The system of clause 24, wherein the hub comprises a first connection interface for connection with the handle, and a second connection interface for connection with the handle, separate from the first connection interface, the first inlet being positioned on the first connection interface, and the second inlet being positioned on the second connection interface.
C34. A system for delivering an agent including a plurality of particles to a target site, the system according to any one or more of clauses 24 to 33.
C3 5. A system for delivering an agent including a plurality of particles to a target site, the system comprising:
   a catheter having a first wall defining an inner lumen for delivery of the agent to the target site, and a second wall surrounding the first wall to define an outer lumen therebetween for delivery of a fluid to the target site, the catheter having a distal end terminating with a primary opening of the inner lumen and a secondary opening of the outer lumen;
   a hub associated with a proximal end of the catheter and comprising a first connection interface having a first inlet for receiving a supply of the agent, and a second connection interface having a second inlet for receiving a supply of the fluid; and,
   a handle for housing a supply of the agent, and a supply of the fluid;
   wherein the catheter and the hub are removably attached to the handle;
   wherein the first connection interface is separate from the second connection interface; and,
   wherein the first inlet is in fluid communication with the inner lumen, and the second inlet is in fluid communication with the outer lumen.
C36. The system of clause 35, wherein the first inlet comprises a male or female threaded connector, and the handle comprises a corresponding mating male or female threaded connector.
C37. The system of clause 35, wherein the second inlet comprises a male or female threaded connector, and the handle comprises a corresponding mating male or female threaded connector.
C3 8. The system of clause 3 5, wherein the first inlet comprises one of a male or female threaded connector, and the second inlet comprises the other of the male or female threaded connector, the handle comprising corresponding mating male or female threaded connectors.
C39. The system of clause 35, wherein a first lumen extends through the hub from the first inlet to a first outlet in fluid communication with the inner lumen of the catheter, the first lumen receiving a supply of the agent form the handle and being axially aligned with the inner lumen.
C40. The system of clause 35, wherein a second lumen extends through the hub from the second inlet to a second outlet in fluid communication with the outer lumen of the catheter, the second lumen receiving a supply of the fluid form the handle, the second lumen at least partially surrounding the first lumen at the second outlet.
C41. A system for delivering an agent including a plurality of particles to a target site, the system according to any one or more of clauses 35 to 40.
C42. A system for delivering an agent including a plurality of particles to a target site, the system comprising:
   a catheter having a first wall defining an inner lumen for delivery of the agent to the target site, and a second wall surrounding the first wall to define an outer lumen therebetween for delivery of a fluid to the target site, the catheter having a distal end terminating with a primary opening of the inner lumen and a secondary opening of the outer lumen; and,
   a hub associated with a proximal end of the catheter and comprising a connection interface having a first inlet for receiving a supply of the agent and a second inlet for receiving a supply of the fluid;
   a handle for housing a supply of the agent, and a supply of the fluid;
   wherein the catheter and the hub are removably attached to the handle; and,
   wherein the first inlet is in fluid communication with the inner lumen, and the second inlet is in fluid communication with the outer lumen.
C43. The system of clause 42, wherein an inner gasket surrounds the first inlet, and the second inlet is positioned outside the gasket.
C44. The system of clause 43, wherein an outer gasket surrounds the second inlet and the inner gasket.
C45. The system of clause 44, wherein the inner gasket and the outer gasket are positioned on the connection interface.
C46. The system of clause 44, wherein the inner gasket and the outer gasket are positioned on the handle.
C47. The system of clause 42, wherein the connection interface comprises a male or female threaded connector, and the handle comprises a corresponding mating male or female threaded connector.
C48. The system of clause 42, wherein a first lumen extends through the hub from the first inlet to a first outlet in fluid communication with the inner lumen of the catheter, the first lumen receiving a supply of the agent form the handle and being axially aligned with the inner lumen.
C49. The system of clause 42, wherein a second lumen extends through the hub from the second inlet to a second outlet in fluid communication with the outer lumen of the catheter, the second lumen receiving a supply of the fluid form the handle, the second lumen at least partially surrounding the first lumen at the second outlet.
C50. A system for delivering an agent including a plurality of particles to a target site, the system according to any one or more of clauses 42 to 49.

It will be understood by those skilled in the art that various other modifications can be made, and equivalents can be substituted, without departing from claimed subject matter. Additionally, many modifications can be made to adapt a particular situation to the teachings of claimed subject matter without departing from the central concept described herein. Therefore, it is intended that claimed subject matter not be limited to the particular embodiments disclosed, but that such claimed subject matter can also include all embodiments falling within the scope of the appended claims, and equivalents thereof.

In the detailed description above, numerous specific details are set forth to provide a thorough understanding of claimed subject matter. However, it will be understood by those skilled in the art that claimed subject matter can be practiced without these specific details. In other instances, methods, devices, or systems that would be known by one of ordinary skill have not been described in detail so as not to obscure claimed subject matter.

Reference throughout this specification to "one embodiment" or "an embodiment" can mean that a particular feature, structure, or characteristic described in connection with a particular embodiment can be included in at least one embodiment of claimed subject matter. Thus, appearances of the phrase "in one embodiment" or "an embodiment" in various places throughout this specification are not necessarily intended to refer to the same embodiment or to any one particular embodiment described. Furthermore, it is to be understood that particular features, structures, or characteristics described can be combined in various ways in one or more embodiments. In general, of course, these and other issues can vary with the particular context of usage. Therefore, the particular context of the description or the usage of these terms can provide helpful guidance regarding inferences to be drawn for that context.

## Claims

1. A system for delivering an agent including a plurality of particles to a target site, the system comprising:
a catheter having a first wall defining an inner lumen for delivery of the agent to the target site, and a second wall surrounding the first wall to define an outer lumen therebetween for delivery of a fluid to the target site, the catheter having a distal end terminating with a primary opening of the inner lumen and a secondary opening of the outer lumen; and,
a hub associated with a proximal end of the catheter and comprising a first inlet for receiving a supply of the agent and a second inlet for receiving a supply of the fluid, wherein the first inlet is in fluid communication with the inner lumen, and the second inlet is in fluid communication with the outer lumen.

2. The system according to claim 1, wherein the hub is fixed to the proximal end of the catheter.

3. The system according to any of the preceding claims, wherein the first inlet comprises a male or female threaded connector.

4. The system according to any of the preceding claims, wherein the second inlet comprises a male or female threaded connector.

5. The system according to any of claims 1 or 2, wherein the first inlet comprises one of a male or female threaded connector, and the second inlet comprises the other of the male or female threaded connector.

6. The system according to any of the preceding claims, wherein a first lumen extends through the hub from the first inlet to a first outlet in fluid communication with the inner lumen of the catheter, the first lumen being axially aligned with the inner lumen.

7. The system according to claim 6, wherein a second lumen extends through the hub from the second inlet to a second outlet in fluid communication with the outer lumen of the catheter, the second lumen at least partially surrounding the first lumen at the second outlet.

8. The system according to claim 7, wherein the hub comprises a connection interface, and wherein the first inlet and the second inlet are formed on the connection interface.

9. The system according to claim 8, wherein a gasket surrounds the first inlet, and the second inlet is positioned outside the gasket.

10. The system according to any of claims 1 to 7, wherein the hub comprises a first connection interface and a second connection interface separate from the first connection interface, the first inlet being positioned on the first connection interface, and the second inlet being positioned on the second connection interface.

11. The system according to claim 1, further comprising:
a handle for housing a supply of the agent, and a supply of the fluid;
wherein the catheter and the hub are removably attached to the handle.

12. The system of claim 11, wherein the first inlet comprises a male or female threaded connector, and the handle comprises a corresponding mating male or female threaded connector.

13. The system of claim 11, wherein the second inlet comprises a male or female threaded connector, and the handle comprising a corresponding mating male or female threaded connector.

14. The system of claim 11, wherein the first inlet comprises one of a male or female threaded connector, and the second inlet comprises the other of the male or female threaded connector, the handle comprising corresponding mating male or female threaded connectors.

15. The system of claim 11, wherein the hub comprises a first connection interface for connection with the handle, and a second connection interface for connection with the handle, separate from the first connection interface, the first inlet being positioned on the first connection interface, and the second inlet being positioned on the second connection interface.
